Europäisches Patentamt

European Patent Office

Office européen des brevets

(19)

(11) Veröffentlichungsnummer: **0 301 369 B1**

# EUROPÄISCHE PATENTSCHRIFT

(12)

(45) Veröffentlichungstag der Patentschrift: **27.11.91**

(51) Int. Cl.5: **A61F 5/01**

(21) Anmeldenummer: **88111569.5**

(22) Anmeldetag: **19.07.88**

(54) **Cervicalstütze.**

(30) Priorität: 28.07.87 DE 3724885
02.01.88 DE 3800022
25.01.88 DE 8800825 U

(43) Veröffentlichungstag der Anmeldung:
**01.02.89 Patentblatt 89/05**

(45) Bekanntmachung des Hinweises auf die
Patenterteilung:
**27.11.91 Patentblatt 91/48**

(84) Benannte Vertragsstaaten:
**AT BE CH DE ES FR GB GR IT LI LU NL SE**

(56) Entgegenhaltungen:
**FR-A- 2 560 518      US-A- 2 820 455**
**US-A- 3 024 784      US-A- 3 504 667**
**US-A- 3 921 626      US-A- 4 194 501**
**US-A- 4 325 363**

(73) Patentinhaber: **Heimann, Dieter, Dr.**
**Königsberger Ring 99**
**W-2340 Kappeln(DE)**

(72) Erfinder: **Heimann, Dieter, Dr.**
**Königsberger Ring 99**
**W-2340 Kappeln(DE)**

(74) Vertreter: **Wehser, Wulf, Dipl.-Ing. et al**
**Patentanwälte Wehser & Fleuchaus Ro-**
**scherstrasse 12**
**W-3000 Hannover 1(DE)**

EP 0 301 369 B1

**Beschreibung**

Die Erfindung betrifft eine Cervicalstütze zum Ruhigstellen der Halswirbelsäule in Form einer den Hals des Patienten Umfassenden gepolsterten Manschette.

Bekannte Cervicalstützen dieser Art (US-A-4,099,523) bestehen aus zwei gegeneinander verschieblichen starren Abschnitten, deren vertikaler Abstand einstellbar ist, um einerseits die Cervicalstütze an verschiedene Körperformen anzupassen und andererseits zur Entlastung der Halswirbelsäule einen gewissen Druck auf die zu stützenden Partien des Kopfes auszuüben.

Nachteilig hierbei ist es, daß durch die bekannten Cervicalstützen der Kopf des Patienten nur insgesamt unterstützt werden kann; eine auf den Einzelfall bezogene partielle Abstützung ist mit der bekannten Cervicalstütze nicht möglich.

Der Erfindung liegt demgemäß die Aufgabe zugrunde, eine Cervicalstütze der eingangs genannten Art so auszubilden, daß mit ihr eine individuelle Ruhigstellung in einem begrenzten Bereich des Kopfes möglich wird, was bei einer Vielzahl von Indikationen, z.B. bei Verschließerscheinungen im Bereich der Halswirbelsäule oder zur postoperativen Behandlung erforderlich ist.

Diese Aufgabe wird erfindungsgemäß dadurch gelöst, daß die Cervicalstütze in ihrem vorderen Bereich mit einer Ausnehmung versehen ist, die zum Ermöglichen einer gewissen Bewegungsfeiheit in der schmerzfreien Richtung einen flexiblen Rahmen aufweist, zwischen dessen oberer und unterer Begrenzung Abstandshalter angeordnet sind, die entlang der Ausnehmung versetzbar sind, so daß die immobilisierenden Bereiche durch Versetzen der Abstandshalter individuall wählbar sind.

Mit dieser Anordnung wird erreicht, daß an den Stellen, an denen sich die Abstandshalter befinden, die Cervicalstütze nicht oder nicht wesentlich eindrückbar ist. Diese immobilisierenden Bereiche können durch Versetzung der Abstandshalter entlang der Ausnehmung individuell gewählt werden. An den Stellen, an denen Abstandshalter nicht vorgesehen sind, ist die Cervicalstütze weitgehend verformbar und gewährleistet mithin eine optimale Bewegungsfreiheit in der jeweils schmerzfreien Richtung.

Es können ein oder mehrere Abstandshalter vorgesehen sein, wobei es zweckmäßig ist, wenn vier Abstandshalter Verwendung finden. Unter Umständen kann es aber bei der erfindungsgemäßen Cervicalstütze schon genügen, daß ein einziger Abstandshalter vorgesehen ist, um die angestrebte Wirkung zu erreichen. Die Abstandshalter können federnd und Feststellbar ausgebildet sein.

Zweckmäßigerweise erstreckt sich die Ausnehmung über einen Umfang der geschlossenen Cervicalstütze von wenigstens 180˚, wobei in Bedarfsfällen die Ausnehmung auch größer gewählt werden kann. Zwischen den beiden einander zugewandten Enden der Ausnehmung liegt der Verschluß der Cervicalstütze, der üblicherweise als Klettverschluß ausgebildet sein kann.

Der flexible Rahmen kann zweckmäßigerweise als flaches Kunststoffband ausgebildet sein, auf dessen Außenseite Polster angebracht sind.

Besonders zweckmäßig ist es, wenn der flexible Rahmen auf seiner Innenseite durch einen umlaufenden federnden Draht ausgesteift ist, an welchem die Abstandshalter angreifen. Dieser Draht kann an dem Rahmen durch ihn übergreifende geschlitzte Haltelaschen verschieblich festgehalten werden, da bei einem Eindrücken der Stütze eine Relativbewegung zwischen Draht und Rahmen auftritt. Außerdem kann der Drahtrahmen im Bereich eines Endes der Ausnehmung offen sein, so daß die so gebildeten freien Enden in Öffnungen des Rahmens einsteckbar sind.

Die Abstandshalter können als Spannschlösser ausgebildet sein, also aus einer Mutter mit zwei gegenläufigen Gewinden und zwei an dem Rahmen angreifenden gegen Drehung gesicherten Schraubbolzen bestehen.

Besonders vorteilhaft ist es jedoch, wenn die Abstandshalter ebenfalls aus einem Federdraht bestehen, da die Cervicalstütze auf diese Weise sehr leicht ausgebildet werden kann. Besonders zweckmäßig ist es hierbei, wenn der Federdraht etwa halbkreisförmig ausgebildet ist und dieser Halbkreis einen Durchmesser hat, welcher der Höhe der Ausnehmung zwischen den Drahtabschnitten in Ruhelage entspricht.

Die Enden des Abstandshalters können mit dem umlaufenden Draht durch Schweißen oder Löten verbunden sein.

Die Abstandshalter können an ihren den Rahmenabschnitten zugewandten Enden mit Öffnungen od. dgl. versehen sein, in die Zapfen od. dgl. an den Rahmenabschnitten eintreten können. Auf diese Weise läßt sich durch einfaches Auseinanderziehen der Rahmenabschnitte der jeweilige Abstandshalter lösen und an eine andere Stelle der Ausnehmung versetzen.

Besonders vorteilhaft ist es hierbei, wenn der den Rahmen aussteifende Draht mit nach innen in Richtung auf die Ausnehmung weisenden Vorsprüngen, Umwinklungen, Ausbuchtungen od. dgl. versehen ist, die im Tragezustand in entsprechende Ausnehmungen der Abstandshalter eingreifen. Zur Lagefixierung können die nach innen weisenden Oberflächen der Umwinklungen mit Rastnuten versehen sein, die mit entsprechenden Widerlagern an den Enden der Abstandshalter zusammenwirken.

Aufgrund der individuellen Einstellbarkeit der erfindungsgemäßen Cervicalstütze ist es auch möglich, diese im Bedarfsfalle so auszugestalten, daß der Kopf völlig ruhiggestellt wird, d.h. eine beidseitige Unbeweglichkeit dadurch gegeben ist, daß auf beiden Seiten des Kopfes bzw. auf einander gegenüberliegenden Seiten der Stütze Abstandshalter angeordnet sind.

Die erfindungsgemäße Ausbildung der Cervicalstütze mit einer solchen relativ großen Ausnehmung hat außerdem den Vorteil, daß im vorderen Halsbereich Druckgefühl und Wärmestau nicht auftreten können. Die Ausnehmung ist hierzu zweckmäßigerweise nicht, wie die übrigen Teile der Cervicalstütze, durch ein Polster sondern durch einen dünnen luftdurchlässigen Bezug abgedeckt.

Die Cervicalstütze kann symmetrisch ausgebildet sein, so daß sie nur umgedreht zu werden braucht, um einen Abstandshalter zu verlagern.

Die Erfindung wird im folgenden anhand von Ausführungsbeispielen in der Zeichnung näher erläutert.

Fig. 1     zeigt in perspektivischer Darstellung eine Ausführungsform der erfindungsgemäßen Cervicalstütze.

Fig. 2     ist eine Draufsicht auf die Cervicalstütze nach Fig. 1 bei einer abgewandelten Ausführungsform.

Fig. 3     zeigt in Abwicklung einen Ausschnitt aus der Ausnehmung der Cervicalstütze nach Fig. 2.

Fig. 4     zeigt eine Abwicklung der Ausnehmung der Cervicalstütze bei einer abgewandelten Ausführungsform.

Fig. 5     ist eine Darstellung der Cervicalstütze ähnlich Fig. 1 bei einer weiteren Ausführungsform.

Fig. 6     zeigt eine Ausführungsform zur lösbaren Halterung von Abstandshaltern zwischen dem oberen und unteren Rahmenabschnitt der Ausnehmung der Cervicalstütze.

Fig. 7     ist eine Darstellung der Cervicalstütze ähnliche den Figuren 1 und 5 bei einer weiteren Ausführungsform.

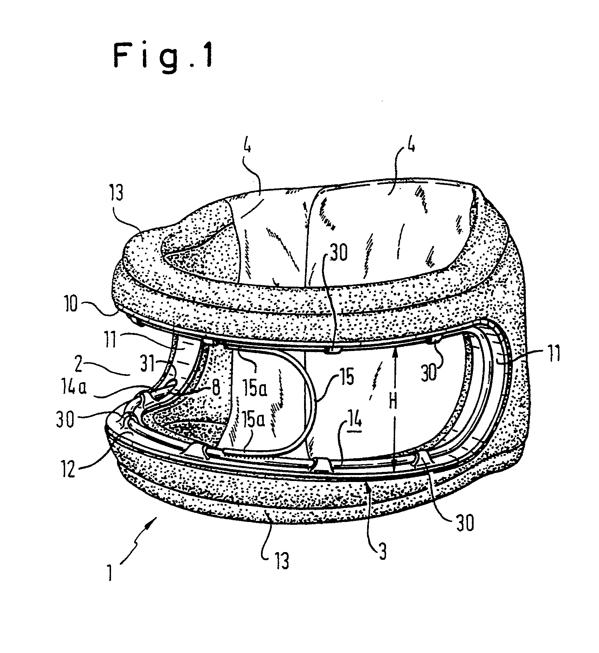

Gemäß Fig. 1 weist die Cervicalstütze 1 eine Ausnehmung 1 auf, die in ihrem im Tragezustand dem vorderen Halsbereich des Trägers zugewandten Bereich angeordnet ist. Die Ausnehmung 2 ist mit einem flexiblen Rahmen 3 versehen, der einen oberen Rahmenabschnitt 10 und einen unteren Rahmenabschnitt 12 aufweist, die im Bereich ihrer Enden durch Bögen 11 miteinander verbunden sind. Bei der in Fig. 1 dargestellten Ausführungsform ist der Rahmen 3 als flacher Kunststoffstreifen ausgebildet.

Die Ausnehmung 2 läßt sich mithin zusammendrücken.

Auf ihrer Ober- bzw. Unterseite sind die Rahmenabschnitte 10 und 12 mit weichen Polstern 13, vorzugsweise aus Schaumstoff, versehen.

An den mit der Ausnehmung 2 versehenen Bereich der Cervicalstütze 1 schließen zwei Verschlußlaschen 4 an, deren einander überlappende und einander zugewandte Flächen jeweils mit einem Abschnitt eines Klettverschlusses versehen sind.

Der flexible Rahmen 3 ist bei der dargestellten Ausführungsform durch einen umlaufenden federnden Draht 14 ausgesteift, an welchem bei der dargestellten Ausführungsform ein Abstandshalter 15 angreift, der aus einem halbkreisförmig gebogenen Draht besteht und im Bereich seiner Angriffsstellen 15a mit dem den Rahmen aussteifenden Draht 14, vorzugsweise durch Löten, verbunden ist.

Der Halbkreis des Abstandshalters 15 hat einen Durchmesser, welcher der Höhe II der Ausnehmung zwischen den Drahtabschnitten in Ruhelage entspricht.

Bei der Ausführungsform nach Fig. 1 ist der Draht 14 durch ihn übergreifende geschlitzte Haltelaschen 30 verschieblich festgehalten, da bei einem Eindrücken der Stütze und damit der Ausnehmung 2 eine Relativbewegung zwischen Draht und Rahmen auftritt.

Der Drahtrahmen 14 kann im Bereich eines Endes der Ausnehmung 2 offen sein, so daß die so gebildeten freien Enden 14a in Öffnungen 31 des Rahmens 3 einsteckbar sind.

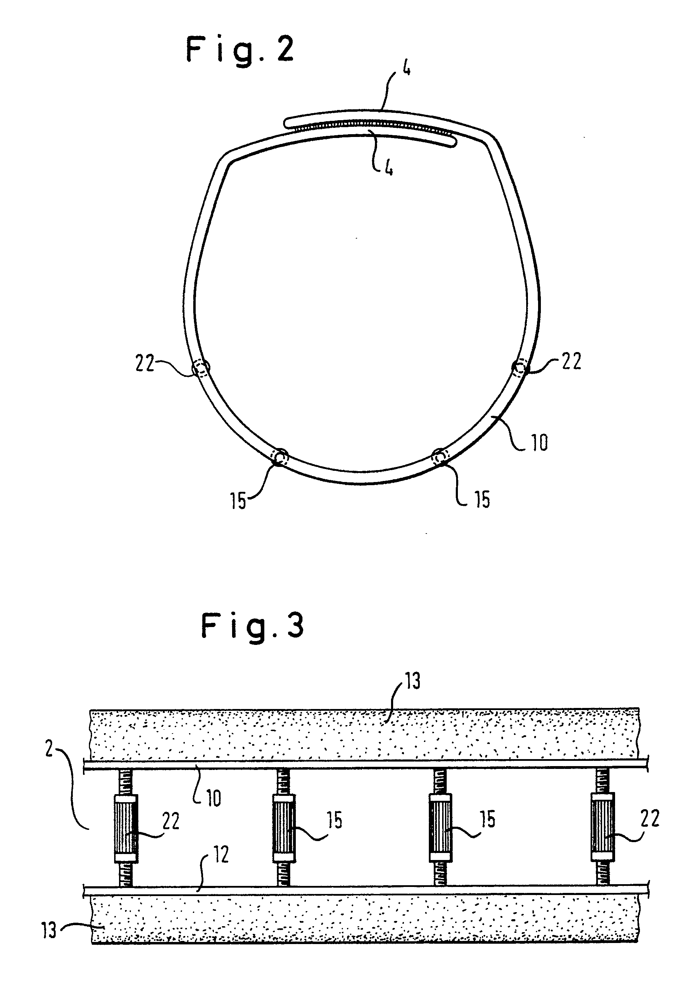

Die Figuren 2 und 3 zeigen eine abgewandelte Ausführungsform, wobei Fig. 2 die Draufsicht auf die Cervicalstütze ist, während Fig. 3 einen Ausschnitt der Ausnehmung 2 wiedergibt. Es sind hier vier Abstandshalter 15 und 22 vorgesehen, die jeweils als Spannschlösser ausgebildet sein können, also aus einer Mutter mit zwei gegenläufigen Gewinden und zwei an dem Rahmen angreifenden gegen Drehung gesicherten Schraubbolzen bestehen können. Die Anordnung der Abstandshalter 15 ist bei der Ausführungsform nach Fig. 3 so gewählt, daß die Abstandshalter 15 in dem Bereich der Kieferwinkel liegen, während die Abstandshalter 22 im Bereich der Warzenfortsätze unter den Ohren liegen. Durch Verstellen der Spannschlösser kann die Cervicalstütze so eingestellt werden, daß der Kopf in der jeweils gewünschten Lage abgestützt wird.

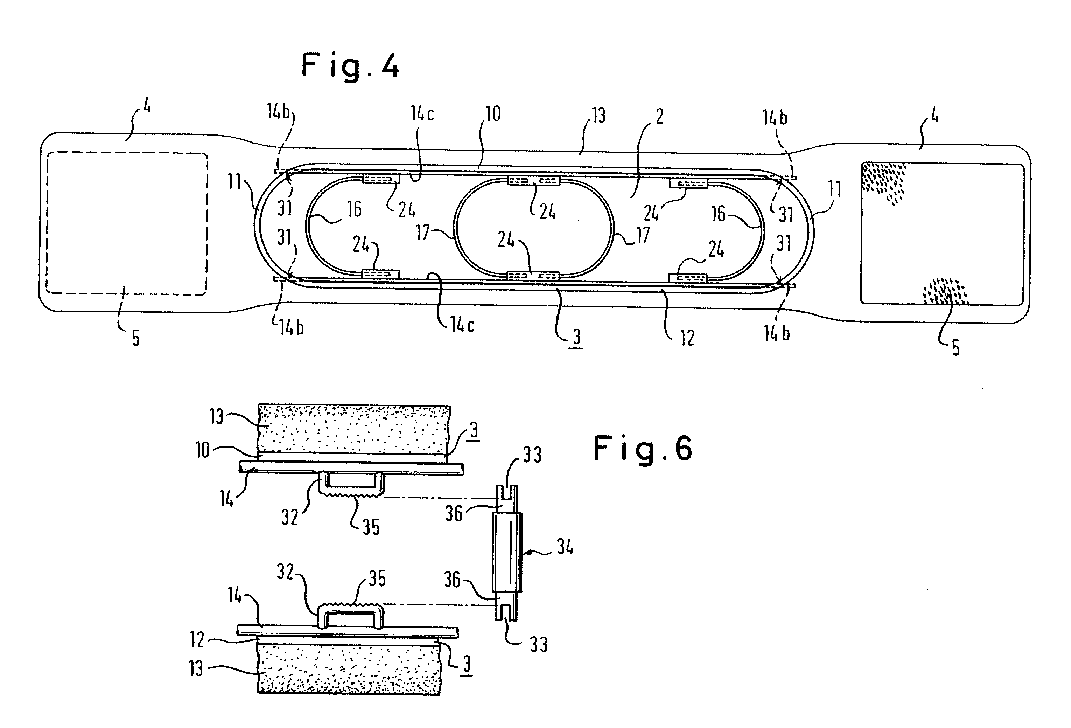

Fig. 4 zeigt die Abwicklung der Ausnehmung 2 der Cervicalstütze bei einer weiteren Ausführungsform. Es sind hier zwei ebenfalls halbkreisförmige aus Federdraht beste hende Abstandshalter 16 im Bereich der Enden der Ausnehmung und zwei weitere Abstandshalter 17 in deren Mitte vorgesehen. Zur Aufnahme dieser Abstandshalter sind Hohlkörper, wie Rohrabschnitte 14 od. dgl. vorgesehen, die aus Kunststoff oder Metall bestehen können und in welche die Enden der Abstandshalter 16 und 17 einsteckbar sind.

Die Anordnung nach Fig. 4 weist also ebenso wie diejenige nach Fig. 3 vier Abstandshalter auf, die bestimmten Stellen des Kopfes zugeordnet sind.

Wie aus Fig. 4 weiter hervorgeht, kann der den flexiblen Rahmen aussteifende Draht 14 aus zwei Einzelstücken 14c bestehen, die entsprechend der Form der Cervicalstütze halbkreisförmig gebogen sind, aber jeweils zwei freie Enden 14b haben, die in entsprechende Öffnungen 31 des Rahmens 3 einsteckbar sind.

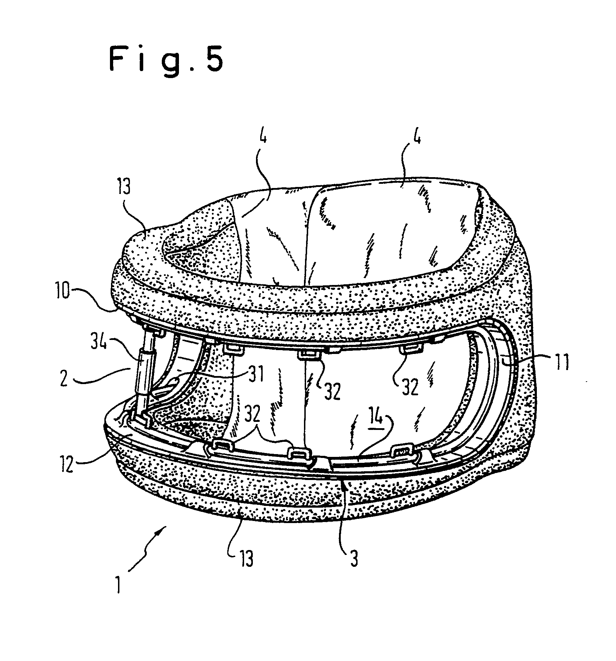

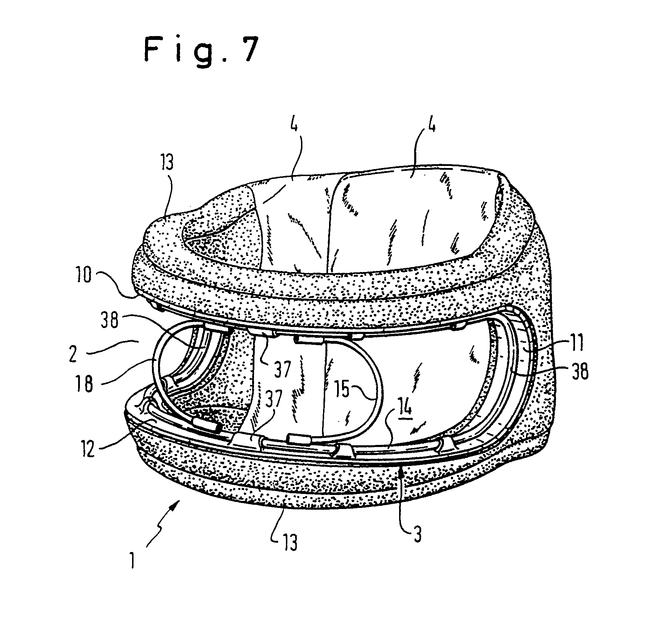

Fig. 5 zeigt in perspektivischer Darstellung eine weitere Ausführungsform der erfindungsgemäßen Cervicalstütze. Bei dieser Ausführungsform ist der den Rahmen 3 aussteifende Draht 14 mit nach innen in Richtung der Ausnehmung 2 weisenden Vorsprüngen, Umwinklungen 32, Ausbuchtungen od. dgl. versehen, die im Tragezustand in entsprechende Ausnehmungen 33 (vgl. Fig. 6) der Abstandshalter 34 eingreifen. Diese Umwinklungen 32 können in Abweichung von der Darstellung nach eine größere Längenausdehnung haben, so daß ein millimetergenaues Einstellen der Lage der Abstandshalter 34 möglich wird. Hierzu können die nach innen weisenden Oberflächen 35 der Umwinklungen 32 mit in entsprechende Widerlager eingreifenden Rastnuten 35 od. dgl. versehen sein.

Fig. 6 verdeutlicht die Ausbildung einer solchen Anordnung. Der jeweilige Abstandshalter 34 ist mit zwei gabelförmigen Enden 36 versehen, die Ausnehmungen 33 aufweisen, welche im Benutzungszustand die Umwinklungen 32 übergreifen und gegen die Rastnuten 35 festlegbar sind. Die Enden 36 können hierbei so ausgebildet sein, daß zusätzlich eine gewisse Klemmkraft auf die Umwinklungen 32 ausgeübt wird.

Fig. 7 schließlich zeigt bei einer Ausführungsform nach Fig. 1 neben dem Abstandshalter 15 einen weiteren Abstandshalter 18. Außerdem geht aus Fig. 7 hervor, daß der umlaufende Draht 14 aus zwei U-förmigen Bügeln besteht, deren freie Enden in Rohrmuffen 37 eingreifen. In entsprechende Rohrmuffen greifen die freien Enden der Abstandshalter 15 und 18 ein (vgl. Fig. 4).

Da mithin im Bereich beider Enden der Ausnehmung 2 Verbindungsbögen 38 angeordnet sind, die sich als solche nicht zusammendrücken lassen und da außerdem im mittleren Bereich die beiden Abstandshalter 15 und 18 vorgesehen sind, läßt sich eine solche Stütze nicht zusammendrücken, so daß sie immobilisierend wirkt. Bei der Ausführungsform nach Fig. 1 dagegen können sich die freien Enden 8 des Federdrahtes 14 gegeneinander bewegen, so daß in diesem Bereich eine Beweglichkeit gegeben ist.

**Patentansprüche**

1. Cervicalstütze zum Ruhigstellen der Halswirbelsäule in Form einer den Hals des Patienten umfassenden gepolsterten Manschette, dadurch gekennzeichnet, daß die Cervicalstütze (1) in ihrem vorderen Bereich mit einer Ausnehmung (2) versehen ist, die zum Ermöglichen einer gewissen Bewegungsfeiheit in der schmerzfreien Richtung einen flexiblen Rahmen (3) aufweist, zwischen dessen oberer und unterer Begrenzung (10,12) Abstandshalter (15) angeordnet sind, die entlang der Ausnehmung (2) versetzbar sind, so daß die immobilisierenden Bereiche durch Versetzen der Abstandshalter individuall wählbar sind.

2. Cervicalstütze nach Anspruch 1, dadurch gekennzeichnet, daß ein oder mehrere Abstandshalter vorgesehen sind.

3. Cervicalstütze nach Anspruch 2, dadurch gekennzeichnet, daß die Abstandshalter federnd und feststellbar ausgebildet sind.

4. Cervicalstütze nach einem der vorhergehenden Ansprüche, dadurch gekennzeichnet, daß sich die Ausnehmung (2) über einen Umfang der geschlossenen Cervicalstütze (1) von wenigstens 180° erstreckt.

5. Cervicalstütze nach Anspruch 4, dadurch gekennzeichnet, daß zwischen den beiden einander zugewandten Enden der Ausnehmung (2) der vorzugsweise als Klettverschluß ausgebildete Verschluß (5) der Cervicalstütze (1) liegt.

6. Cervicalstütze nach einem der vorhergehenden Ansprüche, dadurch gekennzeichnet, daß der flexible Rahmen (3) als flaches Kunststoffband ausgebildet ist, auf dessen Außenseite Polster (13) angebracht sind.

7. Cervicalstütze nach einem der vorhergehenden Ansprüche, dadurch gekennzeichnet, daß der flexible Rahmen (3) auf seiner Innenseite durch einen umlaufenden federnden Draht (14) ausgesteift ist, an welchem die Abstandshalter (15) angreifen.

8. Cervicalstütze nach Anspruch 7, dadurch gekennzeichnet, daß der umlaufende Draht (14) an dem rahmen (3) durch ihn übergreifende geschlitzte Haltelaschen (30) festgehalten wird.

9. Cervicalstütze nach Anspruch 7 oder 8, dadurch gekennzeichnet, daß der Drahtrahmen (14) im Bereich eines Endes der Ausnehmung (2) offen ist, so daß die so gebildeten freien Enden (8) in Öffnungen (31) des Rahmens (3) einsteckbar sind.

10. Cervicalstütze nach einem der vorhergehenden Ansprüche, dadurch gekennzeichnet, daß die Abstandshalter (15,22) als Spannschlösser ausgebildet sind, also aus einer Mutter mit zwei gegenläufigen Gewinden und aus zwei an dem Rahmen (3) angreifenden gegen Drehung gesicherten Schraubbolzen bestehen.

11. Cervicalstütze nach einem der Ansprüche 1 bis 9, dadurch gekennzeichnet, daß die Abstandshalter (15,18) ebenfalls aus einem Federdraht bestehen.

12. Cervicalstütze nach Anspruch 11, dadurch gekennzeichnet, daß der Federdraht etwa halbkreisförmig ausgebildet ist und dieser Halbkreis einen Durchmesser hat, welcher der Höhe (II) der Ausnehmung (2) zwischen den oberen und unteren Abschnitten des Drahtes (14) in Ruhelage entspricht.

13. Cervicalstütze nach Anspruch 11 oder 12, dadurch gekennzeichnet, daß die Enden (15a) des Abstandshalters (15) mit dem umlaufenden Draht (14) durch schweißen oder Löten verbunden sind.

14. Cervicalstütze nach einem der vorhergehenden Ansprüche, dadurch gekennzeichnet, daß die Abstandshalter (15,18,22) an ihren den Abschnitten des Rahmens (3) zugewandten Enden mit Öffnungen od. dgl. versehen sind, in die Zapfen od. dgl. an den Rahmenabschnitten eintreten können.

15. Cervicalstütze nach Anspruch 14, dadurch gekennzeichnet, daß der den Rahmen (3) aussteifende Draht (14) mit nach innen in Richtung auf die Ausnehmung (2) weisenden Vorsprüngen, Umwinklungen (32), Ausbuchtungen od. dgl. versehen ist, die im Tragezustand in entsprechende Ausnehmungen (33) der Abstandshalter (34) eingreifen.

16. Cervicalstütze nach Anspruch 15, dadurch gekennzeichnet, daß zur Lagefixierung die nach innen weisenden Oberflächen der Umwinklungen (32) mit Rastnuten (35) od. dgl. versehen sind, die mit entsprechenden Widerlagern an den Enden (36) der Abstandshalter (34) zusammenwirken.

17. Cervicalstütze nach einem der vorhergehenden Ansprüche, dadurch gekennzeichnet, daß zur Herstellung einer beidseitigen Unbeweglichkeit des Kopfes auf dessen beiden Seiten bzw. auf einander gegenüberliegenden Seiten der Cervicalstütze (1) Abstandshalter (15,18) angeordnet sind.

18. Cervicalstütze nach einem der vorhergehenden Ansprüche, dadurch gekennzeichnet, daß die Ausnehmung (2) ausschließlich durch einen luftdurchlässigen Bezug abgedeckt ist.

19. Cervicalstütze nach einem der vorhergehenden Ansprüche, dadurch gekennzeichnet, daß die Cervicalstütze (1) symmetrisch ausgebildet ist, so daß sie zur Verlagerung eines Abstandshalters nur umgedreht zu werden braucht.

**Claims**

1. Cervical support for holding the cervical vertebral column in the form of a padded collar which surrounds the neck of the patient, characterised in that the cervical support (1) is provided in its front area with an aperture (2) comprising a flexible frame (3) in order to permit a certain amount of freedom of movement in the pain-free direction, and in that spacers (15) are arranged between its upper and lower limits (10, 12) which are transposable along the aperture (2) so that the immobilising areas are individually selectable by way of transposition of the spacers.

2. Cervical support according to claim 1, characterised in that one or more spacers are provided.

3. Cervical support according to claim 2, characterised in that the spacers are constructed to be resilient and lockable.

4. Cervical support according to one of the above claims, characterised in that the aperture (2) extends over a circumference of the closed cervical support (1) of at least 180°.

5. Cervical support according to claim 4, characterised in that the closing means (5) of the cervical support (1), which is preferred to be a barbed hook closing means, is located between the two ends, which face one another, of the aperture (2).

6. Cervical support according to one of the above claims, characterised in that the flexible frame (3) is arranged to be a flat synthetic-material strip with padding (13) at its outside.

7. Cervical support according to one of the above claims, characterised in that the flexible frame (3) is stiffened at its inside by a surrounding resilient wire (14) which is engaged by the spacers (15).

8. Cervical support according to claim 7, characterised in that the surrounding wire (14) is held onto the frame (5) by slotted holding tongues (30) which extend thereover.

9. Cervical support according to claim 7 or 8, characterised in that the wire frame (14) is open in the area of one end of the aperture (2), so that thus formed free ends (8) can be inserted into apertures (31) of the frame (3).

10. Cervical support according to one of the above claims, characterised in that the spacers (15, 22) are constructed to be tension locks, in that they comprise a nut with two counter threads and two rotationally secured threaded bolts which engage the frame (3).

11. Cervical support according to one of claims 1 to 9, characterised in that the spacers (15, 18) are also made of a resilient wire.

12. Cervical support according to claim 11, characterised in that the resilient wire is approximately semi-circular, and this semi-circle has a diameter which corresponds with the height (H) of the aperture (2) between the upper and the lower portions of the wire (14) in the inoperative position.

13. Cervical support according to claim 11 or 12, characterised in that the ends (15a) of the spacer (15) are connected to the surrounding wire (14) by way of welding or soldering.

14. Cervical support according to one of the above claims, characterised in that the spacers (15, 18, 22) have at their ends, which face towards the sections of the frame (3), openings or the like for the insertion of pins or the like at the frame sections.

15. Cervical support according to claim 14, characterised in that the wire (14), which stiffens the frame (3), is provided with protrusions, windings (32), bulges or the like which face inwards in the direction of the aperture (2) and which, when worn, engage respective recesses (33) of the spacers (34).

16. Cervical support according to claim 15, characterised in that the inwards facing surfaces of the windings (32) are provided with holding grooves (35) or the like for fixing the position, which grooves co-act with corresponding abutments at the ends (36) of the spacers (34).

17. Cervical support according to one of the above claims, characterised in that spacers (15, 18) are arranged on both sides, or on opposite sides respectively, of the cervical support (1) for establishing immobility of the head on both sides.

18. Cervical support according to one of the above claims, characterised in that the aperture (2) is covered exclusively by an air-permeable cover.

19. Cervical support according to one of the above claims, characterised in that the cervical support (1) is constructed symmetrically, so that a transposition of a spacer only requires it to be turned round.

**Revendications**

1. Soutien cervical pour immobiliser la colonne vertébrale cervicale se présentant sous la forme d'un manchon rembourré entourant le cou du patient, caractérisé en ce que le soutien cervical (1) est pourvu dans sa région avant d'un évidement (2) qui, pour permettre une certaine liberté de mouvement dans la direction indolore, comporte un cadre flexible (3) entre la délimitation supérieure (10) et la délimitation inférieure (12) duquel sont placés des écarteurs (15) qui sont déplaçables le long de l'évidement (2), de manière que les régions d'immobilisation puissent être choisies individuellement par déplacement des écarteurs.

2. Soutien cervical selon la revendication 1, caractérisé en ce qu'il est prévu un ou plusieurs écarteurs.

3. Soutien cervical selon la revendication 2, caractérisé en ce que les écarteurs sont élastiques et peuvent être bloqués.

4. Soutien cervical selon l'une des revendications précédentes, caractérisé en ce que l'évidement (2) s'étend sur une circonférence d'au moins 180° du soutien cervical (1) fermé.

5. Soutien cervical selon la revendication 4, caractérisé en ce que la fermeture (5) du soutien cervical (1) , conçue de préférence comme une fermeture à ruban à crochets, se situe entre les deux extrémités tournées l'une vers l'autre de l'évidement (2).

6. Soutien cervical selon l'une des revendications précédentes, caractérisé en ce que le cadre (3) flexible est une bande en matière plastique plate sur la face extérieure duquel sont placés des coussins (13).

7. Soutien cervical selon l'une des revendications précédentes, caractérisé en ce que le cadre flexible (3) est rendu rigide sur sa face intérieure par un fil métallique (14) continu, élastique, sur lequel agissent les écarteurs (15).

8. Soutien cervical selon la revendication 7, caractérisé en ce que le fil (14) continu est fixé sur le cadre (3) par des éclisses de maintien (30) fendues, le traversant.

9. Soutien cervical selon la revendication 7 ou 8, caractérisé en ce que le cadre en fil (14) est ouvert dans la région d'une extrémité de l'évidement (2), ce qui fait que les extrémités libres (8) ainsi formées peuvent être insérées dans des ouvertures (31) du cadre (3).

10. Soutien cervical selon l'une des revendications précédentes, caractérisé en ce que les écarteurs (15, 22) sont des tendeurs c'est-à-dire qu'ils sont constitués d'un écrou avec deux filetages en sens contraire et de deux boulons bloqués contre la rotation, agissant sur le cadre (3).

11. Soutien cervical selon l'une des revendications 1 à 9, caractérisé en ce que les écarteurs (15, 18) sont également réalisés dans un fil métallique élastique.

12. Soutien cervical selon la revendication 11, caractérisé en ce que le fil élastique est à peu près en demi-cercle et ce demi-cercle présente un diamètre qui correspond à la hauteur (H) de l'évidement (2) entre les parties supérieures et inférieures du fil (14), en position de repos.

13. Soutien cervical selon la revendication 11 ou 12, caractérisé en ce que les extrémités (15a) de l'écarteur (15) sont assemblées par soudage ou brasage avec le fil continu (14).

14. Soutien cervical selon l'une des revendications précédentes, caractérisé en ce que les écarteurs (15, 18, 22) sont pourvus, à leurs extrémités tournées vers les parties du cadre (3), d'ouvertures ou similaires dans lesquelles peuvent s'engager des tenons ou similaires, des parties de cadre.

15. Soutien cervical selon la revendication 14, caractérisé en ce que le fil (14) renforçant le cadre (3) est pourvu de parties saillantes, de parties coudées (32), de parties bombées ou similaires, dirigées vers l'intérieur en direction de l'évidement (2), qui à l'état d'utilisation, s'engagent dans des évidements (33) correspondants des écarteurs (34).

16. Soutien cervical selon la revendication 15, caractérisé en ce que pour bloquer la position, les surfaces dirigées vers l'intérieur des parties coudées (32) sont pourvues de rainures d'arrêt (35) ou similaires qui coopèrent avec des butées correspondantes prévues sur les extrémités (36) des écarteurs (34).

17. Soutien cervical selon l'une des revendications précédentes, caractérisé en ce que pour réaliser une immobilisation de la tête, des deux côtés, des écarteurs (15, 18) sont placés sur les deux côtés de la tête ou sur des côtés opposés du soutien cervical (1).

18. Soutien cervical selon l'une des revendications précédentes, caractérisé en ce que l'évidement (2) est exclusivement recouvert par un revêtement perméable à l'air.

19. Soutien cervical selon l'une des revendications précédentes, caractérisé en ce que le soutien cervical (1) est symétrique, de sorte qu'il suffit de le retourner pour déplacer un écarteur.

# Fig.1

# Fig. 2

# Fig. 3

# Fig.4

# Fig.6

EP 0 301 369 B1

# Fig.5

# Fig. 7